# FASCICULE DE BREVET EUROPEEN

(11) **EP 1 966 167 B1**
(45) Date de publication et mention de la délivrance du brevet: **16.06.2010**
(21) Numéro de dépôt: 06841899.5
(22) Date de dépôt: 11.12.2006
(51) Int. Cl.: C07D 249/08, C07D 401/12, C07D 405/12, C07D 413/12

(54) **DERIVES DIARYLTRIAZOLMETHYLAMINE, LEUR PREPARATION ET LEUR APPLICATION EN THERAPEUTIQUE.**
DIARYLTRIAZOLMETHYLAMIN-DERIVATE SOWIE IHRE HERSTELLUNG UND THERAPEUTISCHE VERWENDUNG
DIARYLTRIAZOLMETHYLAMINE DERIVATIVES, PREPARATION AND THERAPEUTIC USE THEREOF

(30) Priorité: 12.12.2005 FR 0512713
(43) Date de publication de la demande: 10.09.2008
(73) Titulaire: sanofi-aventis, 75013 Paris (FR)
(72) Inventeur: BARTH, Francis, F-92160 Antony (FR); RINALDI-CARMONA, Murielle, F-92160 Antony (FR)
(74) Mandataire: Kugel, Dominique
(86) Numéro de dépôt international: PCT/FR2006/002694
(87) Numéro de publication internationale: WO 2007/068814

(56) Documents cités:
- WO-A-2004/026301
- WO-A-2006/074445
- DYCK B ET AL: "POTENT IMIDAZOLE AND TRIAZOLE CB1 RECEPTOR ANTAGONISTS RELATED TO SR141716" BIOORGANIC & MEDICINAL CHEMISTRY LETTERS, OXFORD, GB, vol. 14, 2004, pages 1151-1154, XP001197288 ISSN: 0960-894X cité dans la demande
- LANGE J H M ET AL: "BIOISOSTERIC REPLACEMENTS OF THE PYRAZOLE MOIETY OF RIMONABANT: SYNTHESIS, BIOLOGICAL PROPERTIES, AND MOLECULAR MODELING INVESTIGATIONS OF THIAZOLES, TRIAZOLES, AND IMIDAZOLES AS POTENT AND SELECTIVE CB1 CANNABINOID RECEPTOR ANTAGONISTS" JOURNAL OF MEDICINAL CHEMISTRY, AMERICAN CHEMICAL SOCIETY. WASHINGTON, US, vol. 48, 2005, pages 1823-1838, XP002339942 ISSN: 0022-2623
- JAGEROVIC, NADINE ET AL: "Discovery of 5-(4-Chlorophenyl)-1-(2,4-dichlorophenyl)- 3-hexyl-1H-1,2,4- triazole, a Novel in Vivo Cannabinoid Antagonist Containing a 1,2,4-Triazole Motif" JOURNAL OF MEDICINAL CHEMISTRY , 47(11), 2939-2942 CODEN: JMCMAR; ISSN: 0022-2623, 2004, XP002400692
- DATABASE CA [Online] CHEMICAL ABSTRACTS SERVICE, COLUMBUS, OHIO, US; BROWNE, E. J. ET AL: "Triazoles. VI. 1,5 Diaryl 1,2,4 triazole 3 carboxaldehydes" XP002400689 extrait de STN Database accession no. 1962:403989 & JOURNAL OF THE CHEMICAL SOCIETY 575-83 CODEN: JCSOA9; ISSN: 0368-1769, 1962,

## Description

La présente invention a pour objet des dérivés diaryltriazolméthylamine, leur préparation et leur application en thérapeutique.

Des dérivés de 1,5-diaryl-1,2,4-triazole-3-carboxamide sont décrits en tant que modulateurs des récepteurs aux cannabinoïdes dans la demande de brevet WO 2004/026 301 et dans Bioorg. Med. Chem. Lett., 2004,14, p.1151.

La demande de brevet WO 2006/074 445 décrit différents composés hétérocycliques, notamment des dérivés de triazole comme présentant une activité sur les récepteurs CB₁ et CB₂ aux cannabinoïdes.

On a maintenant trouvé des nouveaux dérivés diaryltriazolméthylamine qui possèdent des propriétés antagonistes des récepteurs CB₁ des cannabinoïdes.

La présente invention a pour objet des composés répondant à la formule : 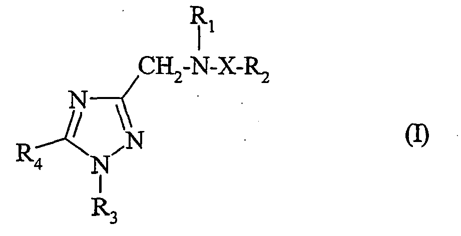 dans laquelle :
- X représente un groupe 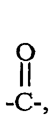 -SO₂-, 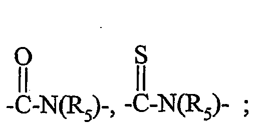
- R₁ représente un atome d'hydrogène ou un groupe (C₁-C₄)alkyle ;
- R₂ représente :
   - un (C₁-C₇)alkyle non substitué ou substitué par un groupe trifluorométhyle ou par un groupement CO₂R₆ ou CONR₇R₈ ;
   - un radical carbocyclique non aromatique en C₃-C₁₂ non substitué ou substitué une ou plusieurs fois par un (C₁-C₄)alkyle ;
   - un méthyle substitué par un radical carbocyclique non aromatique en C₃-C₁₂ et non substitué ou substitué une ou plusieurs fois sur le carbocycle par un (C₁-C₄)alkyle ;
   - un phényle non substitué ou substitué une ou plusieurs fois par des substituants choisis indépendamment parmi un atome d'halogène, un (C₁-C₄)alkyle, un hydroxyle, un (C₁-C₄)alcoxy, un cyano, un groupe trifluorométhyle, un groupe trifluorométhoxy, un groupement CO₂R₆ ou CONR₇R₈ ; ou parmi un radical phényle, phénoxy, pyrrolyle, imidazolyle, pyridyle ou pyrazolyle, lesdits radicaux étant non substitués ou substitués une ou plusieurs fois par un (C₁-C₄)alkyle ;
   - un phénoxyméthyle non substitué sur le méthyle, ou substitué sur le méthyle par un ou deux groupes (C₁-C₄)alkyle, et non substitué sur le phényle, ou substitué sur le phényle par un ou plusieurs substituants choisis indépendamment parmi un atome d'halogène, un (C₁-C₄)alkyle, un (C₁-C₄)alcoxy, un groupe trifluorométhyle, un groupe trifluorométhoxy, un groupement CO₂R₆ ou CONR₇R₈ ;
   - un benzyle non substitué ou substitué une ou plusieurs fois sur le phényle par des substituants choisis indépendamment parmi un atome d'halogène, un cyano, un (C₁-C₄)alkyle, un (C₁-C₄)alcoxy, un groupe trifluorométhyle, un groupe trifluorométhoxy, un groupement CO₂R₆ ou CONR₇R₈ ; ou substitué en alpha par un ou deux groupes semblables ou différents choisis parmi un (C₁-C₄)alkyle ou un (C₃-C₇)cycloalkyle ;
   - un benzhydryle, un groupe benzhydrylméthyle ;
   - un 1,2,3,4-tétrahydronaphtalènyle non substitué ou substitué une ou plusieurs fois par un (C₁-C₄)alkyle ;
   - un radical pyrrolyle, imidazolyle, pyridyle, pyrazolyle, furyle, thiényle, lesdits radicaux étant non substitués ou substitués par un ou plusieurs substituants choisis indépendamment parmi un atome d'halogène, un (C₁-C₄)alkyle ou un groupe trifluorométhyle;
   - un indolyle non substitué ou substitué une ou plusieurs fois par des substituants choisis indépendamment parmi un atome d'halogène ou un (C₁-C₄) alkyle;
   - un benzofuryle non substitué ou substitué une ou plusieurs fois par des substituants choisis indépendamment parmi un atome d'halogène ou un (C₁-C₄)alkyle ;
   - un 2,1,3-benzoxadiazolyle ;
- R₃ représente un phényle non substitué ou substitué une ou plusieurs fois par des substituants choisis indépendamment parmi un atome d'halogène, un (C₁-C₄)alkyle, un (C₁-C₄)alcoxy, un groupe trifluorométhyle, trifluorométhoxy, ou un groupement S(O)ₙAlk ;
- R₄ représente un phényle non substitué ou substitué une ou plusieurs fois par des substituants choisis indépendamment parmi un atome d'halogène, un (C₁-C₄)alkyle, un (C₁-C₄)alcoxy, un groupe trifluorométhyle, trifluorométhoxy, ou un groupement S(O)ₙAlk ;
- R₅ représente un atome d'hydrogène ou un (C₁-C₄)alkyle ;
- R₆ représente un C₁-C₄ alkyle ;
- R₇ et R₈ représentent chacun indépendamment un atome d'hydrogène ou un (C₁-C₄)alkyle ;
- n représente 0, 1 ou 2 ;
- Alk représente un (C₁-C₄)alkyle;
   à la condition que lorsque X représente un groupe -CO- ou -CONR₅- , R₂ soit différent de :
   - un radical carbocyclique non aromatique en (C₄-C₇) non substitué ou substitué une ou plusieurs fois par un (C₁-C₄)alkyle ;
   - un phényle non substitué ou substitué une ou plusieurs fois par des substituants choisis indépendamment parmi un atome d'halogène, un (C₁-C₄)alkyle, un hydroxyle, un (C₁-C₄)alcoxy, un cyano, un groupe trifluorométhyle, un groupement CO₂R₆ ou CONR₇R₈ ; ou parmi un radical pyrrol-1-yle ou pyrazol-1-yle, lesdits radicaux étant non substitués ou substitués une ou plusieurs fois par un (C₁-C₄)alkyle ;
   - un 1,2,3,4-tétrahydronaphtalènyle non substitué ou substitué une ou plusieurs fois par un (C₁-C₄)alkyle ;
   - un radical pyrrolyle, imidazolyle, pyridyle, pyrazolyle, furyle, thiényle, lesdits radicaux étant non substitués ou substitués par un ou plusieurs substituants choisis indépendamment parmi un atome d'halogène, un (C₁-C₄)alkyle ou un groupe trifluorométhyle ;
   - un indolyle non substitué ou substitué une ou plusieurs fois par des substituants choisis indépendamment parmi un atome d'halogène ou un (C₁-C₄) alkyle ;
   - un benzofuryle non substitué ou substitué une ou plusieurs fois par des substituants choisis indépendamment parmi un atome d'halogène ou un (C₁-C₄)alkyle ;
   - un 2,1,3-benzoxadiazolyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₄)alkyle ou respectivement (C₁-C₇)alkyle, on entend un radical alkyle linéaire ou ramifié de un à quatre atomes de carbone ou respectivement de un à sept atomes de carbone, tel que le radical méthyle, éthyle, propyle, isopropyle, butyle, isobutyle, *sec*-butyle, *tert*-butyle, pentyle, isopentyle, hexyle, isohexyle, heptyle.

Par (C₁-C₄)alcoxy on entend un radical alcoxy linéaire ou ramifié de un à quatre atomes de carbone tel que le radical méthoxy, éthoxy, propoxy, isopropoxy, butoxy, *sec*-butoxy, *tert*-butoxy.

Par un radical carbocyclique non aromatique en C₃-C₁₂ on entend : un radical monocyclique ou un radical di- ou tricyclique condensé ou ponté ; par radical monocyclique on entend un cycloalkyle, par exemple cyclopropyle, cyclobutyle, cyclopentyle, cyclohexyle, cycloheptyle, les radicaux cyclopentyle, cyclohexyle et cycloheptyle étant préférés ; par radical di- ou tricyclique condensé ou ponté, on entend par exemple le bicyclo[2.2.1]heptyle, le bicyclo[2.2.2]octyle, le bicyclo[3.2.1]octyle, l'adamantyle.

Parmi les composés de formule (I), objets de l'invention, on distingue :
- les composés de formule (IA) dans laquelle -X- représente un groupe -CO- et les substituants R₁ à R₄ sont tels que définis pour les composés de formule (I) ;
- les composés de formule (IB) dans laquelle -X- représente un groupe -SO₂- et les substituants R₁ à R₄ sont tels que définis pour les composés de formule (I) ;
- les composés de formule (IC) dans laquelle -X- représente un groupe -CON(R₅)- et les substituants R₁ à R₅ sont tels que définis pour les composés de formule (I) ;
- les composés de formule (ID) dans laquelle -X- représente un groupe -CSN(R₅)- et les substituants R₁ à R₅ sont tels que définis pour les composés de formule (I).

Parmi les composés objets de l'invention, on préfère les composés de formule (I) dans laquelle :
- R₁ représente un atome d'hydrogène ;
- R₂ a l'une des valeurs définies pour (I) ;
- R₃ et R₄ représentent un 2,4-dichlorophényle et un 4-chlorophényle, un 4-chlorophényle et un 2,4-dichlorophényle, un 2,4-dichlorophényle et un 4-méthoxyphényle ;
ainsi que leurs hydrates ou leurs solvats.

On préfère tout particulièrement les composés de formule (I) dans laquelle les substituants R₁, R₃, et R₄ sont tels que définis ci-dessus et R₂ représente un groupe benzhydrylméthyle ou benzyhydryle ; ainsi que leurs hydrates ou leurs solvats.

On préfère également les composés de formule (IB) et (ID) dans laquelle les substituants Rₗ₁, R₃ et R₄ sont tels que définis ci-dessus et R₂ représente un groupe choisi parmi :
- un phényle substitué une ou plusieurs fois par des substituants choisis indépendamment parmi un atome d'halogène, un (C₁-C₄)alkyle, un groupe trifluorométhyle ou un groupe trifluorométhoxy ;
- un groupe benzhydrylméthyle ou benzyhydryle ;
- un radical indol-2-yle non substitué ou substitué par un (C₁-C₄)alkyle ; ainsi que leurs hydrates ou leurs solvats.

Conformément à l'invention, on peut préparer les composés de formule (I) selon un procédé qui est **caractérisé en ce que** :
on traite un composé de formule : 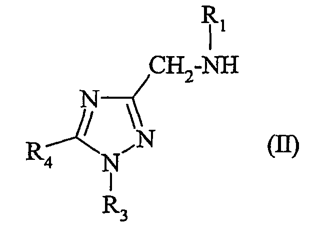 dans laquelle R₁, R₃, et R₄ sont tels que définis pour un composé de formule (I) :
   - soit par un acide ou un dérivé fonctionnel de cet acide de formule :

      HOOC-R₂ (III)

      dans laquelle R₂ est tel que défini pour un composé de formule (I), lorsqu'on doit préparer un composé de formule (IA) dans laquelle -X- représente un groupe -CO- ;
   - soit par un halogénure de sulfonyle de formule :

      Hal-SO₂-R₂ (IV)

      dans laquelle R₂ est tel que défini pour un composé de formule (I) et Hal représente un atome d'halogène, préférentiellement le chlore, lorsqu'on doit préparer un composé de formule (IB) dans laquelle -X- représente un groupe -SO₂- ;
   - soit par un halogénoformiate de formule :

      HalCOOAr (V)

      dans laquelle Hal représente un atome d'halogène et Ar représente un phényle ou un 4-nitrophényle pour obtenir un composé intermédiaire de formule : 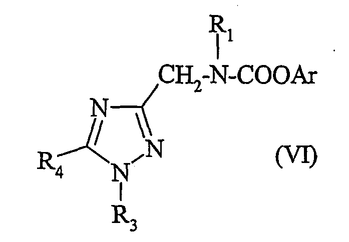 dans laquelle R₁, R₃, et R₄ sont tels que définis pour un composé de formule (I), que l'on fait réagir ensuite avec une amine de formule :

      HN(R₅)R₂ (VII)

      dans laquelle R₂ et R₅ sont tels que définis pour un composé de formule (I), lorsqu'on doit préparer un composé de formule (IC) dans laquelle -X- représente un groupe -CON(R₅)- ;
   - soit par un isothiocyanate R₂N=C=S (VIII), lorsque l'on doit préparer un composé de formule (ID) dans laquelle -X- représente un groupe -CSNH-.

Le cas échéant, on peut préparer un composé de formule (IC) ou (ID) dans lequel R₅ représente un groupe (C₁-C₄)alkyle par une réaction d'alkylation sur le composé correspondant de formule (I) dans laquelle R₅ représente un atome d'hydrogène.

Eventuellement, on transforme le composé de formule (I) en l'un de ses sels d'addition à un acide.

Lorsqu'on traite un composé de formule (II) avec l'acide de formule (III) lui-même, on opère en présence d'un agent de couplage utilisé en chimie peptidique tel que le 1,3-dicyclohexylcarbodiimide (DCC) ou l'hexafluorophosphate de benzotriazol-1-yloxytris(diméthylamino) phosphonium (BOP) ou l'hexafluorophosphate de benzotriazol-1-yloxytris(pyrrolidino) phosphonium (PyBOP) ou le tétrafluoroborate de 2-(1*H*-benzotriazol-1-yl)-1,1,3,3-tétraméthyl uronium (TBTU), en présence d'une base telle que la triéthylamine, la N,N-diisopropyléthylamine ou la 4-diméthylaminopyridine, dans un solvant tel que le dichlorométhane, le dichloroéthane, le N-N-diméthylformanide ou le tétrahydrofurane à une température comprise entre - 10°C et la température de reflux du solvant.

Comme dérivé fonctionnel de l'acide (III) on peut utiliser le chlorure d'acide, l'anhydride, un anhydride mixte, un ester alkylique en C₁-C₄ dans lequel l'alkyle est droit ou ramifié, un ester activé, par exemple l'ester de p-nitrophényle.

Ainsi dans le procédé selon l'invention, on peut aussi faire réagir le chlorure de l'acide obtenu par réaction du chlorure de thionyle ou du chlorure d'oxalyle sur l'acide de formule (III), avec le composé de formule (II), dans un solvant, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer l'anhydride mixte de l'acide de formule (III) par réaction du chloroformiate d'éthyle avec l'acide de formule (III), en présence d'une base telle que la triéthylamine, et à le faire réagir avec le composé de formule (II), dans un solvant tel que le dichlorométhane, sous une atmosphère inerte, à la température ambiante, en présence d'une base telle que la triéthylamine.

Lorsqu'on traite un composé de formule (II) avec un halogénure de sulfonyle de formule (IV), on opère en présence d'une base telle que la triéthylamine ou la diisopropyléthylamine, dans un solvant tel que le dichlorométhane ou le tétrahydrofurane et à une température comprise entre la température ambiante et la température de reflux du solvant.

Lorsqu'on traite un composé de formule (II) avec un halogénoformiate de formule (V), on opère en présence d'une base telle que la triéthylamine, dans un solvant tel que le dichlorométhane et à une température comprise entre 0°C et la température ambiante. Puis on fait réagir le composé intermédiaire de formule (VI) ainsi obtenu avec une amine de formule (VII), dans un solvant tel que le dichlorométhane, en présence d'une base telle que la triéthylamine et à une température comprise entre 0°C et la température de reflux du solvant.

Selon une variante du procédé on peut préparer les composés de formule (IC) dans laquelle -X- représente un groupe -CON(R₅)- dans lequel R₅ = H par réaction d'un composé de formule (II) avec un isocyanate de formule R₂-N=C=O (VIII), en présence d'une base telle que la triéthylamine, dans un solvant tel que le dichlorométhane et à une température comprise entre la température ambiante et la température de reflux du solvant.

Selon une autre variante du procédé on peut préparer les composés de formule (IC) dans laquelle -X- représente un groupe -CON(R₅)- par réaction d'un composé de formule (II) avec un composé de formule ClCON(R₅)R₂ (IX) en présence d'une base telle que la triéthylamine, dans un solvant tel que le dichlorométhane et à une température comprise entre 0°C et la température ambiante.

Les composés de formule (IA), (IB), (IC avec R₅ ≠ H), (ID avec R₅ ≠ H) dans lesquelles R₁ représente un (C₁-C₄)alkyle peuvent aussi être préparés à partir des composés correspondant de formule (I) dans laquelle R₁ représente un atome d'hydrogène par une méthode choisie parmi les méthodes connues de l'homme de l'art, telle que l'alkylation par un halogénure d'alkyle.

Les composés de formule (I) ainsi obtenus peuvent être ultérieurement séparés du milieu réactionnel et purifiés selon les méthodes classiques, par exemple par cristallisation ou chromatographie.

Les composé de formule (II) peuvent être préparés selon le schéma réactionnel suivant :

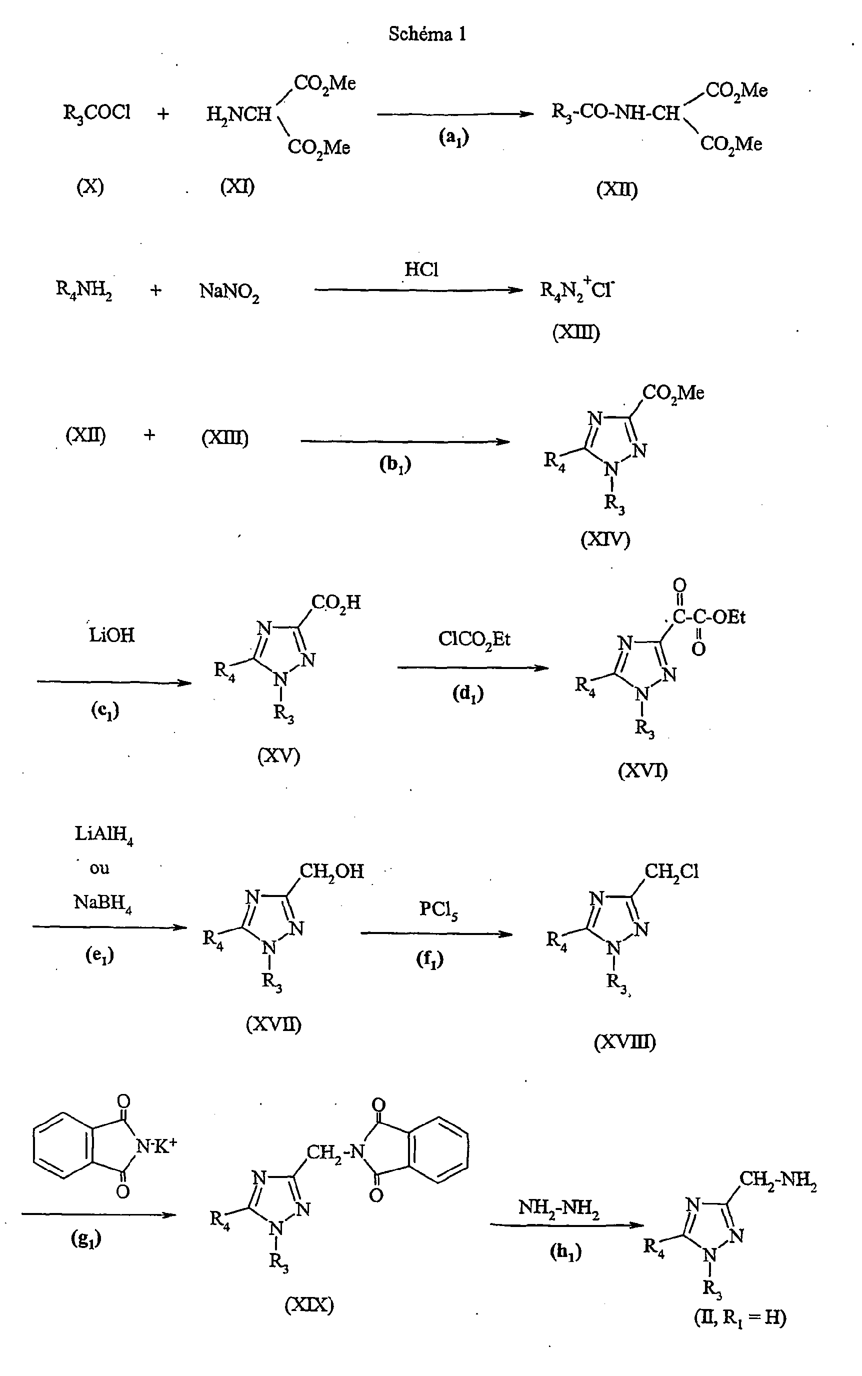

A l'étape a₁, on prépare le malonate de formule (XII) par action du chlorure d'acide R₃COCI (X) sur le diméthylamino malonate (XI). On prépare ensuite le chlorure de diazonium de formule (XIII) que l'on fait agir sur le malonate de formule (XI) pour obtenir à l'étape b₁ lé dérivé d'ester de l'acide 1,5-diphényl-1*H*-1,2,4-triazole-3-carboxylique. A l'étape b₁, l'ester est hydrolysé, par exemple par LiOH, puis à l'étape d₁, on fait agir le chloroformiate d'éthyle pour préparer le composé de formule (XVI). Ce composé est réduit à l'étape c₁, par exemple par un agent réducteur tel que LiAlH₄ ou NaBH₄ et l'alcool obtenu de formule (XVII) est traité par un agent de chloration tel que PCl₅. On fait ensuite agir le phtalimide de potassium (étape g₁) puis l'hydrate d'hydrazine (étape h₁) pour préparer le composé de formule (II).

Le cas échéant, on peut transformer un composé de formule (II) dans laquelle R₁ représente un atome d'hydrogène en composé de formule (II) dans laquelle R₁ est un (C₁-C₄)alkyle en utilisant des méthodes connues de l'homme de l'art, telles que par exemple, la réaction avec un agent alkylant, l' amination réductrice par un aldéhyde en milieu réducteur, ou encore l'acylation par un chlorure d'acide suivi d'une réduction.

De manière préférentielle, les oxazoles de formule (II) sont préparés selon le schéma réactionnel suivant :
Les exemples suivants décrivent la préparation de certains composés conformes à l'invention. Les exemples ne sont pas limitatifs et ne font qu'illustrer la présente invention. Dans les Préparations et les Exemples on utilise les abréviations suivantes :
   DMF : N,N-diméthylformamide
   THF : tétrahydrofurane
   DCM : dichlorométhane
   MeOH : méthanol
   TFA : acide trifluoroacétique
   AcOH : acide acétique
   DBU : 1,8-diazabicyclo[5,4,0]undec-7-ène
   DCC : 1,3-dicyclohexylcarbodimide
   DIPEA : diisopropyléthylamine
   BOP : benzotriazol-1-yloxyfris(diméthylamino)phosphonium hexafluorophosphate
   PyBOP : benzotriazol-1-yloxytris(pyrrolidino)phosphonium hexafluorophosphate
   TBTU : tétrafluoroborate de 2-(*1H*-benzotriazol-1-yl)-1,1,3,3-tétraméthyluronium
   Rdt : rendement
   TA : température ambiante
   HPLC ou CLHP : chromatographie liquide haute performance

Les composés selon l'invention sont analysés par couplage LC/UV/MS (chromatographie liquide / détection UV / spectrométrie de masse). On mesure les pics moléculaires (MH⁺) et le temps de rétention (t) en minutes.

### Conditions MS2

On utilise une colonne XTerra MS C18 de 2,1 x 30 mm, 3,5 µm, à 30°C, débit 0,8 ml/minute.

L'éluant est composé comme suit :
- solvant A : 0,025 % d'acide trifluoroacétique (TFA) dans l'eau ;
- solvant B : 0,025 % de TFA dans l'acétonitrile.

### Gradient :

| Temps (mn) | % A | % B |
|---|---|---|
| 0 | 100 | 0 |
| 2 | 0 | 100 |
| 2,7 | 0 | 100 |
| 2,75 | 100 | 0 |

La détection UV est effectuée par un détecteur à barette de diodes entre 2.10 et 400 nm et la détection de masse en mode ionisation chimique ESI positif.

### Conditions MS5

On utilise une colonne XTerra MS C18 de 2,1 x 30 mm, 3,5 µm, débit 1 ml/minute.

L'éluant est composé comme suit :
- solvant A : 0,025 % de TFA dans l'eau,
- solvant B : 0,025 % de TFA dans l'acétonitrile.

### Gradient :

| Temps (mn) | % A | % B |
|---|---|---|
| 0 | 100 | 0 |
| 2 | 0 | 100 |
| 2,7 | 0 | 100 |
| 2,75 | 100 | 0 |

La détection UV est effectuée par un détecteur à barette de diodes entre 210 et 400 nm et la détection de masse en mode ESI positif.

### Méthode A :

On utilise une colonne Symmetry C18 de 2,1 x 50 mm, 3,5 µm, à 30°C, débit 0,4 ml/minute.

L'éluant est composé comme suit :
- solvant A : 0,005 % d'acide trifluoroacétique (TFA) dans l'eau à pH 3,15 ;
- solvant B : 0,005 % de TFA dans l'acétonitrile.

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 210 nM et la détection de masse en mode, ionisation chimique ESI positif.

### PREPARATIONS

### 1. Préparations des composés de formule (II).

### Préparation 1.1

### 1-(1-(4-Chlorophényl)-5-(2,4-dichlorophényl)-1H-1,2,4-triazol-3-yl)méthanamine.

### A) ((2,4-Dichlorobenzoyl)amino)malonate de diméthyle.

A une solution de 5g de diméthylamino malonate dans le DCM (60ml), est ajouté 13,2ml de triéthylamine. Après 30 minutes d'agitation à température ambiante, 5,2g de 2,4-dichlorobenzoyle est additionné lentement au milieu réactionnel. L'ensemble est agité à température ambiante pendant 2 heures. Le milieu est ensuite dilué au DCM (50ml) et lavé successivement par une solution aqueuse HCI (3N), puis à l'eau. La phase organique est séchée sur Na₂SO₄, filtrée et évaporée à sec conduisant au produit brut attendu (Rdt = 90%).

### B) Ester méthylique de l'acide 1-(4-chlorophényl)-5-(2,4-dichlorophényl)-1H-1,2,4-triazole-3-carboxylique.

A une solution préalablement préparée contenant 3,25g de 4-chloroaniline, 15ml d'acide acétique et 5ml d'HCI concentré, est additionné lentement à froid 1,9g de NaNO₂ en solution dans 15ml d'eau. Le milieu réactionnel est agité pendant 30 minutes. Cette solution hétérogène est ajoutée lentement à froid à un mélange préalablement réalisé contenant 7,4g du composé préparé à l'étape précédente, 6,3g d'acétate de sodium dans 40ml de méthanol. L'ensemble est agité à température ambiante pendant 2 heures. Le milieu hétérogène est filtré. Le filtrat est évaporé à sec et un mélange méthanol-eau est additionné. Un nouveau précipité formé est récupéré par filtration. L'ensemble des précipités est placé dans 50ml de méthanol et traité par une solution de MeONa/MeOH (150mg de Na dans 20ml de MeOH). L'ensemble est agité à température ambiante pendant 1 heure et évaporé à sec. Le produit brut obtenu est directement impliqué dans l'étape suivante.

### C) Acide 1-(4-chlorophényl)-5-(2,4-dichlorophényl)-1H-1,2,4-triazole-3-carboxylique.

Le brut réactionnel obtenu à l'étape précédente est placé dans un mélange THF-H₂O (155ml/55ml), puis 1,15g de LiOH dilué dans un minimum d'eau est additionné à 0°C. Après 1 heure d'agitation à O°C, le milieu réactionnel est lavé à l'ether. La phase aqueuse est acidifiée (∼pH1) par une solution de KHSO₄. L'ensemble est extrait à l'acétate d'éthyle. La phase organique est séchée sur NaSO₄, filtrée et évaporée à sec pour donner le composé attendu. (Rdt = 79%).

### D) (1-(4-Chlorophényl)-5-(2,4-dichlorophényl)-1H-1,2,4-triazol-3-yl)methanol.

A une solution de 6,7g du dérivé d'acide obtenu à l'étape précédente dans le THF (4,5ml), est ajouté à 0°C 3ml de Et₃N puis 1,9ml de chloroformiate d'éthyle. Après 15 minutes d'agitation à 0°C, 2,0g de NaBH₄ est additionné. Le méthanol est ensuite additionné goutte à goutte. Après 30 minutes d'agitation à 0°C, l'ensemble est hydrolysé par addition d'une solution aqueuse de HCl (1N). L'ensemble est évaporé à sec puis est repris dans le THF (120ml) et 0,8g de LiAlH₄ est additionné par portion toutes les 30 minutes. L'ensemble est hydrolysé de manière classique et filtré sur silice pour donner le composé attendu. (Rdt = 63%).

### E) 3-(Chlorométhyl)-1-(4-chlorophényl)-5-(2,4-dichlorophényl)-1H-1,2,4-triazole.

A une solution de 4g de dérivé alcool préparé à l'étape précédente dans 75ml de DCM, est ajouté à 0°C 4,7g de PCl₅ par portion. L'ensemble est agité à température ambiante pendant 4 heures et demie. Le milieu réactionnel est versé dans un mélange eau-glace. L'ensemble est extrait au DCM. La phase organique est lavée par une solution saturée de NaCl. La phase organique est séchée sur Na₂SO₄, filtrée et évaporée à sec, conduisant au produit attendu. (Rdt = 97%).

### F) 2-((1-(4-Chlorophényl)-5-(2,4-dichlorophényl)-1H-1,2,4-triazol-3-yl)méthyl)-1H-isoindole-1,3-(2H)-dione.

A une solution de 4,1g de dérivé chloré obtenu à l'étape précédente dans le DMF (30ml), est additionné 1,6 g de NaI puis 2,2g de 1,3-dioxo-1,3-dihydroisoindol-2-ide de potassium. L'ensemble est chauffé à 65°C pendant 3 heures. Le milieu réactionnel est refroidi à température ambiante. Le milieu est dilué à l'acétate d'éthyle et lavé successivement par une solution aqueuse de NaOH (0,2N) puis à l'eau. L'ensemble hétérogène est filtré. La phase organique est séchée sur Na₂SO₄, filtrée et évaporée à sec. Le produit attendu est présent dans le précipité et dans le filtrat évaporé avec un rendement total de 87%.

### G) 1-(1-(4-Chlorophényl)-5-(2,4-dichlorophényl)-1H-1,2,4-triazol-3-yl)méthanamine.

A une solution de 2,2g de dérivé phtalimide obtenu à l'étape précédente dans 30ml d'éthanol, est additionné 1,9ml d'hydrate d'hydrazine. L'ensemble est chauffé au reflux du solvant pendant 1 heure. Le milieu hétérogène est refroidi à température ambiante et filtré. Le précipité est rincé à l'éthanol. Le filtrat est évaporé à sec, repris à l'acétate d'éthyle puis lavé plusieurs fois par une solution aqueuse NaOH (0,2N). La phase organique est séchée sur Na₂SO₄, filtrée et évaporée à sec. Le produit attendu est isolé par formation de son chlorydrate dans l'ether éthylique-HCl. (Rdt = 70%).

D'autres intermédiaires de formule (II), ont été préparés selon le mode opératoire décrit ci-dessus :

**TABLEAU 1**

| | | | |
|---|---|---|---|
| 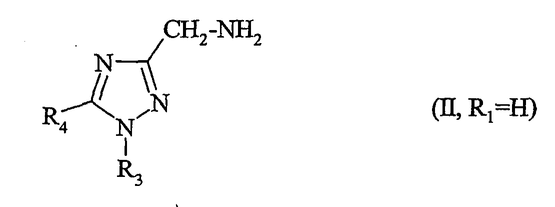 | | | |

| **Préparation N°** | **R3** | **R4** | **Caractérisation (conditions)** |
|---|---|---|---|
| Préparation 1.1 | 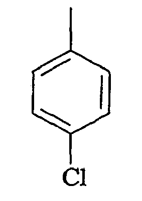 | 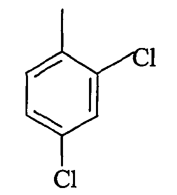 | MH⁺ = 352,9 t = 6,56 (A) |
| Préparation 1.2 | 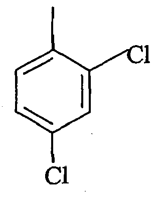 | 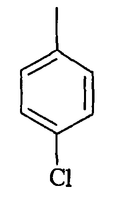 | MH⁺ = 352,9 t = 6,55 (A) |
| Préparation 1.3 | 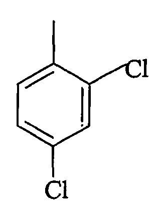 | 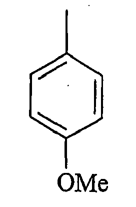 | MH⁺ = 348,9 t=6,16 (A) |

### EXEMPLE 1 :

Les composés de formule (I) dans laquelle -X- = -CO- sont préparés par chimie combinatoire selon le procédé décrit ci-après :

On dissout les acides carboxyliques de formule (III) dans le DMF à la concentration de 0,25 M en présence de 3 équivalents de DIPEA. Dans chaque puits de 2 ml on place 120 µl de ces solutions et ajoute 120 ml d'une solution de TBTU dans le DMF à la concentration de 0,25 M. On ajoute dans chaque puits 300 µl d'une solution contenant la méthylamine de formule (II) dans le DMF à la concentration de 0,1 M et 3 équivalents de DIPEA. On agite les plaques à TA pendant 16 heures puis évapore. On dissout les produits formés dans chaque puits par 500 µl d'AcOEt, puis on ajoute 400 µl de Na₂CO₃ 0,1 M et agite les plaques. Après décantation, on écarte 430 µl de phase aqueuse, puis on ajoute 300 µl de NaCl à 5 % et on agite les plaques. On écarte ensuite 350 µl de phase aqueuse par LC/UV/MS et évapore le reste sous vide pour obtenir les composés attendus.

### EXEMPLE 2 :

Les composés de formule (I) dans laquelle -X- = -CONH- sont préparés par chimie combinatoire selon le procédé décrit ci-après :

On dissout les composés de formule (II) dans le DMF à la concentration de 0,1. M en présence de 3 équivalents de DIPEA. Dans chaque puit de 2 ml on place 300 µl de ces solutions et ajoute 120 µl d'une solution contenant le composé isocyanate de formule (VIII) dans le THF à la concentration de 0,25 M. On agite les plaques à TA pendant 16 heures. On dissout les produits formés dans chaque puit par ajout de 500 µl d'AcOEt, ajoute 400 µl de Na₂CO₃ 0,1 M et agite les plaques. Après décantation on écarte 430 µl de phase aqueuse, ajoute 300 µl de NaCl à 5 % et agite les plaques. Après décantation, on écarte 350 µl de phase aqueuse, prélève 20µl pour analyse par LC/UV/MS et évapore le reste sous vide pour obtenir les composés attendus.

### EXEMPLE 3 :

Les composés de formule (I) dans laquelle -X- = -SO₂- sont préparés par chimie combinatoire selon le procédé décrit ci-après :

On dissout les composés de formule (II) dans le DMF à la concentration de 0,1 M en présence de 3 équivalents de DIPEA. Dans chaque puits de 2 ml on place 300 µl de ces solutions et ajoute 120 µl d'une solution contenant le chlorure de sulfonyle de formule (IV) correspondant dans le THF à la concentration de 0,25 M. On agite les plaques à TA pendant 16 heures puis évapore. On dissout les produits formés dans chaque puits par ajout de 500 µl d'AcOEt, ajoute 400 µl de Na₂CO₃ 0,1 M et agite les plaques. Après décantation on écarte 430 µl de phase aqueuse, ajoute 300 µl de NaCl à 5 % et agite les plaques. Après décantation, on écarte 350 µl de phase aqueuse, prélève 20µl pour analyse par LC/UV/MS et évapore le reste sous vide pour obtenir les composés attendus.

Les tableaux qui suivent illustrent les structures chimiques et les propriétés physiques de quelques composés selon l'invention. Dans ces tableaux Me, Et, nPr, tBu, illustrent respectivement les groupes méthyle, éthyle, n-propyle, *tert*-butyle.

**TABLEAU III**

| | | | | | |
|---|---|---|---|---|---|
| 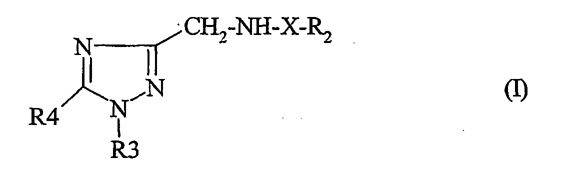 | | | | | |

| **Composés N°** | **R₂** | **X** | **R₃** | **R₄** | **Caractérisation Conditions** |
|---|---|---|---|---|---|
| 1 | 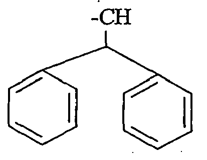 | -CONH- | 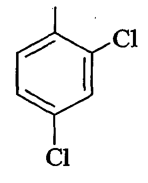 | 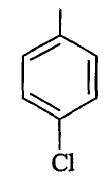 | MH⁺ = 562,5 t = 1,92 MS 2 |
| 2 | 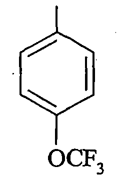 | -CO- | 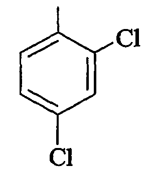 | 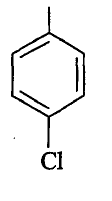 | MH⁺ = 541,4 t = 1,93 MS 2 |
| 3 | 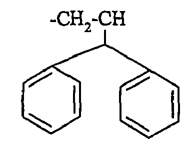 | -CO- | 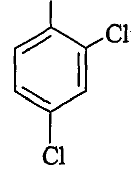 | 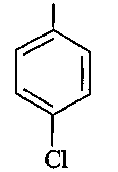 | MH⁺ = 561,5 t = 1,95 MS 2 |
| 4 | 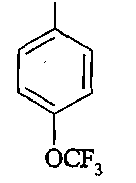 | -CO- | 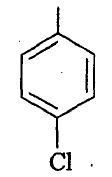 | 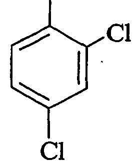 | MH⁺ = 540,8 t = 2,38 MS 5 |
| 5 | 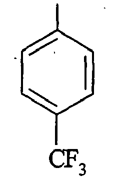 | -C(S)NH- | 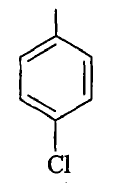 | 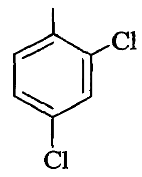 | MH⁺ = 555,8 t = 2,00 MS 5 |
| 6 | 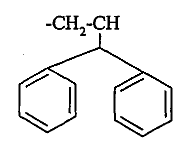 | -CO- | 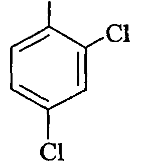 | 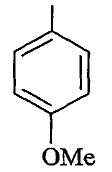 | MH⁺ = 557,6 t = 1,86 MS 2 |
| 7 | 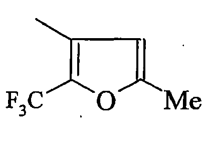 | -SO₂- | 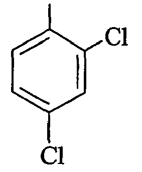 | 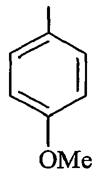 | MH⁺ = 560,8 t=2,31 MS 5 |
| 8 | 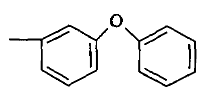 | -CO- | 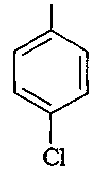 | 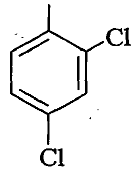 | MH⁺ = 548,8 t = 2,42 MS 5 |
| 9 | 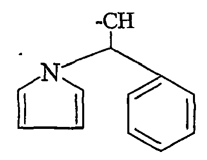 | -CO- | 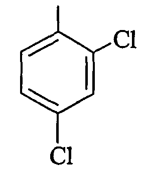 | 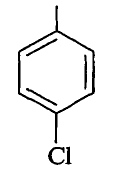 | MH⁺ = 535,8 t = 2,35 MS 5 |
| 10 | 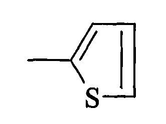 | -SO₂- | 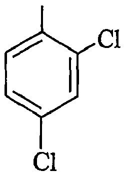 | 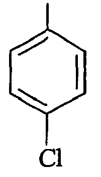 | MH⁺ = 499,4 t = 1,82 MS 2 |
| 11 | 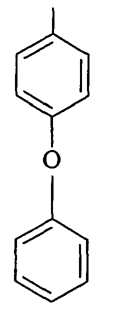 | -CO- | 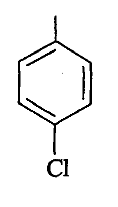 | 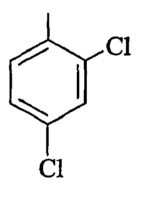 | MH⁺ = 548,8 t = 2,44 MS 5 |
| 12 | 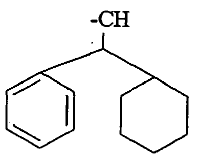 | -CO- | 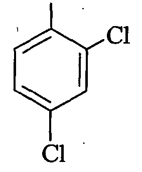 | 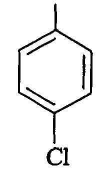 | MH⁺ = 553,6 t = 2,06 MS 2 |
| 13 | 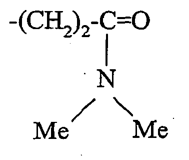 | -CO- | 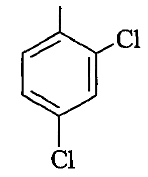 | 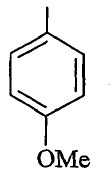 | MH⁺ = 476,6 t = 1,48 MS 2 |
| 14 | 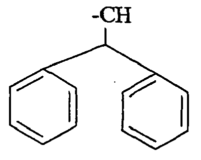 | -CONH- | 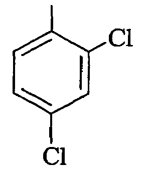 | 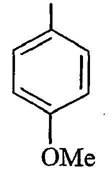 | MH⁺ = 557,9 t = 2,27 MS 5 |
| 15 | 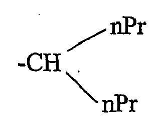 | -CO- | 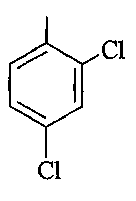 | 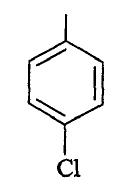 | MH⁺ = 479,6 t =1,94 MS 2 |
| 16 | 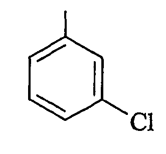 | -SO₂- | 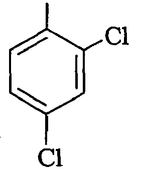 | 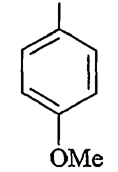 | MH⁺= 522,8 t = 2,25 MS 5 |
| 17 | 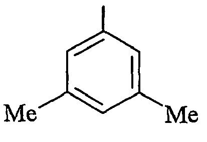 | -SO₂- | 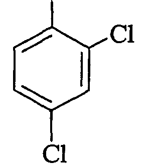 | 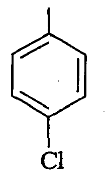 | MH⁺ = 521,5 t =1,93 MS2 |
| 18 | 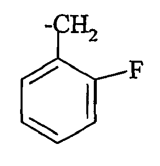 | -CONH- | 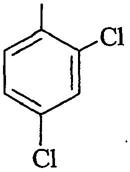 | 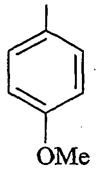 | MH⁺ = 499,9 t = 2,13 MS 5 |

Les composés de formule (I) possèdent une très bonne affinité *in vitro* (IC₅₀ ≤ 5.10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

La toxicité des composés de formule (I) est compatible avec leur utilisation en tant que médicament.

Ainsi, selon un autre de ses aspects, l'invention a pour objet des médicaments pour la médecine humaine ou vétérinaire qui comprennent un composé de formule (I), ou encore un solvat ou un hydrate du composé de formule (I).

Ainsi les composés selon l'invention peuvent être utilisés chez l'homme ou chez l'animal, dans le traitement ou la prévention de maladies impliquant les récepteurs aux cannabinoïdes CB₁.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et de l'hyperactivité (TDAH) chez les enfants hyperkinétiques (MBD) ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire.

Les composés de formule (I) selon l'invention peuvent être utilisés comme médicaments dans la médecine humaine ou vétérinaire dans le traitement des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardio-vasculaires. De plus, les composés de formule (I) selon l'invention peuvent être utilisés en tant que médicaments dans le traitement des troubles gastro-intestinaux, des troubles diarrhéiques, des ulcères, des vomissements, des troubles vésicaux et urinaires, des troubles d'origine endocrinienne, des troubles cardio-vasculaires, de l'hypotension, du choc hémorragique, du choc septique, de la cirrhose chronique du foie, de la stéatose hépatique, de la stéatohépatite, de l'asthme, du syndrome de Raynaud, du glaucome, des troubles de la fertilité, de l'interruption de grossesse, de l'accouchement prématuré, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose.

Selon la présente invention, les composés de formule (I) sont tout particulièrement utiles pour le traitement des troubles psychotiques, en particulier la schizophrénie, les troubles de l'attention et de l'hyperactivité (TDAH) chez les enfants hyperkinétiques (MBD) ; pour le traitement des troubles de l'appétit et de l'obésité ; pour le traitement des déficits mnésiques et cognitifs ; pour le traitement de la dépendance alcoolique, de la dépendance nicotinique, c'est à dire pour le sevrage alcoolique et pour le sevrage tabagique.

Plus particulièrement, les composés de formule (I) selon la présente invention sont utiles dans le traitement et la prévention des troubles de l'appétit, des troubles métaboliques, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), et de ses solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un.patient, d'une dose efficace d'un composé selon l'invention, ou hydrates ou solvats.

## Revendications

1. Composé répondant à la formule : 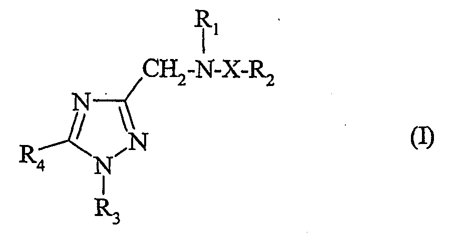 dans laquelle :
- X représente un groupe 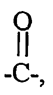 -SO₂-, 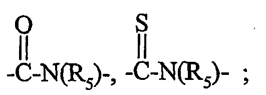
- R₁ représente un atome d'hydrogène ou un groupe (C₁-C₄)alkyle ;
- R₂ représente :
. un (C₁-C₇)alkyle non substitué ou substitué par un groupe trifluorométhyle ou par un groupement CO₂R₆ ou CONR₇R₈ ;
. un radical carbocyclique non aromatique en C₃-C₁₂ non substitué ou substitué une ou plusieurs fois par un (C₁-C₄)alkyle ;
. un méthyle substitué par un radical carbocyclique non aromatique en C₃-C₁₂ et non substitué ou substitué une ou plusieurs fois sur le carbocycle par un (C₁-C₄)alkyle ;
. un phényle non substitué ou substitué une ou plusieurs fois par des substituants choisis indépendamment parmi un atome d'halogène, un (C₁-C₄)alkyle, un hydroxyle, un (C₁-C₄)alcoxy, un cyano, un groupe trifluorométhyle, un groupe trifluorométhoxy, un groupement CO₂R₆ ou CONR₇R₈ ; ou parmi un radical phényle, phénoxy, pyrrolyle, imidazolyle, pyridyle ou pyrazolyle, lesdits radicaux étant non substitués ou substitués une ou plusieurs fois par un (C₁-C₄)alkyle ;
. un phénoxyméthyle non substitué sur le méthyle, ou substitué sur le méthyle par un ou deux groupes (C₁-C₄)alkyle, et non substitué sur le phényle, ou substitué sur le phényle par un ou plusieurs substituants choisis indépendamment parmi un atome d'halogène, un (C₁-C₄)alkyle, un (C₁-C₄)alcoxy, un groupe trifluorométhyle, un groupe trifluorométhoxy, un groupement CO₂R₆ ou CONR₇R₈ ;
. un benzyle non substitué ou substitué une ou plusieurs fois sur le phényle par des substituants choisis indépendamment parmi un atome d'halogène, un cyano, un (C₁-C₄)alkyle, un (C₁-C₄)alcoxy, un groupe trifluorométhyle, un groupe trifluorométhoxy, un groupement CO₂R₆ ou CONR₇R₈ ; ou substitué en alpha par un ou deux groupes semblables ou différents choisis parmi un (C₁-C₄)alkyle ou un (C₃-C₇)cycloalkyle;
. un benzhydryle, un groupe benzhydrylméthyle ;
. un 1,2,3,4-tétrahydronaphtalènyle non substitué ou substitué une ou plusieurs fois par un (C₁-C₄)alkyle ;
. un radical pyrrolyle, imidazolyle, pyridyle, pyrazolyle, furyle, thiényle, lesdits radicaux étant non substitués ou substitués par un ou plusieurs substituants choisis indépendamment parmi un atome d'halogène, un (C₁-C₄)alkyle ou un groupe trifluorométhyle;
. un indolyle non substitué ou substitué une ou plusieurs fois par des substituants choisis indépendamment parmi un atome d'halogène ou un (C₁-C₄) alkyle;
. un benzofuryle non substitué ou substitué une ou plusieurs fois par des substituants choisis indépendamment parmi un atome d'halogène ou un (C₁-C₄)alkyle ;
. un 2,1,3-benzoxadiazolyle ;
- R₃ représente un phényle non substitué ou substitué une ou plusieurs fois par des substituants choisis indépendamment parmi un atome d'halogène, un (C₁-C₄)alkyle, un (C₁-C₄)alcoxy, un groupe trifluorométhyle, trifluorométhoxy, ou un groupement S(O)ₙAlk ;
- R₄ représente un phényle non substitué ou substitué une ou plusieurs fois par des substituants choisis indépendamment parmi un atome d'halogène, un (C₁-C₄)alkyle, un (C₁-C₄)alcoxy, un groupe trifluorométhyle, trifluorométhoxy, ou un groupement S(O)ₙAlk ;
- R₅ représente un atome d'hydrogène ou un (C₁-C₄)alkyle ;
- R₆ représente un C₁-C₄ alkyle ;
- R₇ et R₈ représentent chacun indépendamment un atome d'hydrogène ou un (C₁-C₄)alkyle ;
- n représente 0, 1 ou 2 ;
- Alk représente un (C₁-C₄)alkyle ;
à la condition que lorsque X représente un groupe -CO- ou -CONR₅- , R₂ soit différent de :
. un radical carbocyclique non aromatique en (C₄-C₇) non substitué ou substitué une ou plusieurs fois par un (C₁-C₄)alkyle ;
. un phényle non substitué ou substitué une ou plusieurs fois par des substituants choisis indépendamment parmi un atome d'halogène, un (C₁-C₄)alkyle, un hydroxyle, un (C₁-C₄)alcoxy, un cyano, un groupe trifluorométhyle, un groupement CO₂R₆ ou CONR₇R₈ ; ou parmi un radical pyrrol-1-yle ou pyrazol-1-yle, lesdits radicaux étant non substitués ou substitués une ou plusieurs fois par un (C₁-C₄)alkyle ;
. un 1,2,3,4-tétrahydronaphtalènyle non substitué ou substitué une ou plusieurs fois par un (C₁-C₄)alkyle ;
. un radical pyrrolyle, imidazolyle, pyridyle, pyrazolyle, furyle, thiényle, lesdits radicaux étant non substitués ou substitués par un ou plusieurs substituants choisis indépendamment parmi un atome d'halogène, un (C₁-C₄)alkyle ou un groupe trifluorométhyle ;
. un indolyle non substitué ou substitué une ou plusieurs fois par des substituants choisis indépendamment parmi un atome d'halogène ou un (C₁-C₄) alkyle ;
. un benzofuryle non substitué ou substitué une ou plusieurs fois par des substituants choisis indépendamment parmi un atome d'halogène ou un (C₁-C₄)alkyle;
. un 2,1,3-benzoxadiazolyle ;
à l'état de base ou de sels d'addition à des acides ou à l'état d'hydrate ou de solvats.

2. Composé selon la revendication 1, de formule (IA) dans laquelle -X- représente un groupe -CO- et les substituants R1 à R4 sont tels que définis pour les composés de formule (I) ;
à l'état de base ou de sels d'addition à des acides ou à l'état d'hydrate ou de solvats.

3. Composé selon la revendication 1, de formule (IB) dans laquelle -X- représente un groupe -SO₂- et les substituants R₁ à R₄ sont tels que définis pour les composés de formule (I) ;
à l'état de base ou de sels d'addition à des acides ou à l'état d'hydrate ou de solvats.

4. Composé selon la revendication 1, de formule (IC) dans laquelle -X- représente un groupe -CON(R₅)- et les substituants R₁ à R₅ sont tels que définis pour les composés de formule (I) ;
à l'état de base ou de sels d'addition à des acides ou à l'état d'hydrate ou de solvats.

5. Composé selon la revendication 1, de formule (ID) dans laquelle -X- représente un groupe -CSN(R₅)- et les substituants R₁ à R₅ sont tels que définis pour les composés de formule (I) ;
à l'état de base ou de sels d'addition à des acides ou à l'état d'hydrate ou de solvats.

6. Procédé de préparation d'un composé de formule (I) selon la revendication 1 **caractérisé en ce que** :
on traite un composé de formule : 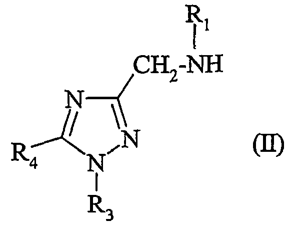 dans laquelle R₁, R₃, et R₄ sont tels que définis pour un composé de formule (I) à la revendication 1 :
- soit par un acide ou un dérivé fonctionnel de cet acide de formule :
HOOC-R₂ (III)
dans laquelle R₂ est tel que défini pour un composé de formule (I) à la revendication 1, lorsqu'on doit préparer un composé de formule (IA) dans laquelle
- X- représente un groupe -CO- ;
- soit par un halogénure de sulfonyle de formule :
Hal-SO₂-R₂ (IV)
dans laquelle R₂ est tel que défini pour un composé de formule (I) à la revendication 1 et Hal représente un atome d'halogène, préférentiellement le chlore, lorsqu'on doit préparer un composé de formule (IB) dans laquelle -X-représente un groupe -SO₂- ;
- soit par un halogénoformiate de formule :
HalCOOAr (V)
dans laquelle Hal représente un atome d'halogène et Ar représente un phényle ou un 4-nitrophényle pour obtenir un composé intermédiaire de formule : 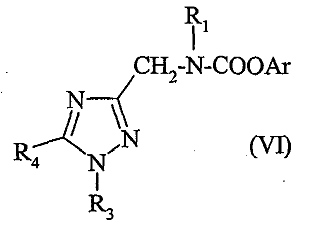 dans laquelle R₁, R₃, et R₄ sont tels que définis pour un composé de formule (I) à la revendication 1, que l'on fait réagir ensuite avec une amine de formule :
HN(R₅)R₂ (VII)
dans laquelle R₂ et R₅ sont tels que définis pour un composé de formule (I) à la revendication 1, lorsqu'on doit préparer un composé de formule (IC) dans laquelle
-X- représente un groupe -CON(R₅)- ;
- soit par un isothiocyanate R₂N=C=S (VIII), lorsque l'on doit préparer un composé de formule (ID) dans laquelle X- représente un groupe -CSNH-.

7. Médicament **caractérisé en ce qu'**il comprend un composé de formule (I) selon l'un quelconque des revendications 1 à 5, ou un hydrate ou un solvat d'un composé de formule (I).

8. Composition pharmaceutique, **caractérisée en ce qu'**elle comprend un composé de formule (I) selon l'une quelconque des revendications 1 à 5, ou un hydrate ou un solvat de ce composé, ainsi qu'au moins un excipient pharmaceutiquement acceptable.

9. Utilisation d'un composé de formule (I) selon l'une quelconque des revendications 1 à 5 pour la préparation d'un médicament destiné au traitement et à la prévention des troubles de l'appétit, des troubles métaboliques, des troubles gastro-intestinaux, des phénomènes inflammatoires, des maladies du système immunitaire, des troubles psychotiques, de la dépendance alcoolique, de la dépendance nicotinique.

## Claims

1. Compound corresponding to the formula: 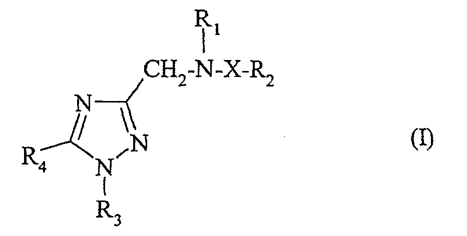 in which:
- X represents a group 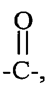 -SO₂-, 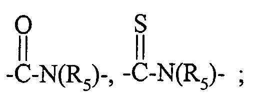
- R₁ represents a hydrogen atom or a (C₁-C₄)alkyl group;
- R₂ represents:
• a (C₁-C₇)alkyl, which is unsubstituted or substituted with a trifluoromethyl group or with a group CO₂R₆ or CONR₇R₈;
• a C₃-C₁₂ non-aromatic carbocyclic radical, which is unsubstituted or substituted one or more times with a (C₁-C₄)alkyl;
• a methyl substituted with a C₃-C₁₂ non-aromatic carbocyclic radical that is unsubstituted or substituted one or more times on the carbocycle with a (C₁-C₄)alkyl;
• a phenyl, which is unsubstituted or substituted one or more times with substituents independently chosen from a halogen atom, a (C₁-C₄)alkyl, a hydroxyl, a (C₁-C₄)alkoxy, a cyano, a trifluoromethyl group, a trifluoromethoxy group, a group CO₂R₆ or CONR₇R₈; or from a phenyl, phenoxy, pyrrolyl, imidazolyl, pyridyl or pyrazolyl radical, the said radicals being unsubstituted or substituted one or more times with a (C₁-C₄)alkyl;
• a phenoxymethyl, which is unsubstituted on the methyl or substituted on the methyl with one or two (C₁-C₄)alkyl groups, and unsubstituted on the phenyl or substituted on the phenyl with one or more substituents independently chosen from a halogen atom, a (C₁-C₄)alkyl, a (C₁-C₄)alkoxy, a trifluoromethyl group, a trifluoromethoxy group and a group CO₂R₆ or CONR₇R₈;
• a benzyl, which is unsubstituted or substituted one or more times on the phenyl with substituents independently chosen from a halogen atom, a cyano, a (C₁-C₄)alkyl, a (C₁-C₄)alkoxy, a trifluoromethyl group, a trifluoromethoxy group and a group CO₂R₆ or CONR₇R₈; or α-substituted with one or two identical or different groups chosen from a (C₁-C₄)alkyl and a (C₃-C₇)cycloalkyl;
• a benzyhydryl or a benzhydrylmethyl group;
• a 1,2,3,4-tetrahydronaphthyl, which is unsubstituted or substituted one or more times with a (C₁-C₄)alkyl;
• a pyrrolyl, imidazolyl, pyridyl, pyrazolyl, furyl or thienyl radical, the said radicals being unsubstituted or substituted with one or more substituents independently chosen from a halogen atom, a (C₁-C₄)alkyl and a trifluoromethyl group;
• an indolyl, which is unsubstituted or substituted one or more times with substituents independently chosen from a halogen atom and a (C₁-C₄)alkyl;
• a benzofuryl, which is unsubstituted or substituted one or more times with substituents independently chosen from a halogen atom and a (C₁-C₄)alkyl;
• a 2,1,3-benzoxadiazolyl;
- R₃ represents a phenyl, which is unsubstituted or substituted one or more times with substituents independently chosen from a halogen atom, a (C₁-C₄)alkyl, a (C₁-C₄)alkoxy, a trifluoromethyl or trifluoromethoxy group and a group S(O)ₙAlk;
- R₄ represents a phenyl, which is unsubstituted or substituted one or more times with substituents independently chosen from a halogen atom, a (C₁-C₄)alkyl, a (C₁-C₄)alkoxy, a trifluoromethyl or trifluoromethoxy group, and a group S(O)ₙAlk;
- R₅ represents a hydrogen atom or a (C₁-C₄)alkyl;
- R₆ represents a C₁-C₄ alkyl;
- R₇ and R₈ represent each independently a hydrogen atom or a (C₁-C₄)alkyl;
- n represents 0, 1 or 2;
- Alk represents a (C₁-C₄)alkyl;
on condition that when X represents a group -CO- or - CONR₅-, R₂ is other than:
• a C₄-C₇ non-aromatic carbocyclic radical, which is unsubstituted or substituted one or more times with a (C₁-C₄)alkyl;
• a phenyl, which is unsubstituted or substituted one or more times with substituents independently chosen from a halogen atom, a (C₁-C₄)alkyl, a hydroxyl, a (C₁-C₄)alkoxy, a cyano, a trifluoromethyl group and a group CO₂R₆ or CONR₇R₈; or from a 1-pyrrolyl or 1-pyrazoyl radical, the said radicals being unsubstituted or substituted one or more times with a (C₁-C₄)alkyl;
• a 1,2,3,4-tetrahydronaphthyl, which is unsubstituted or substituted one or more times with a (C₁-C₄)alkyl;
• a pyrrolyl, imidazolyl, pyridyl, pyrazolyl, furyl or thienyl radical, the said radicals being unsubstituted or substituted with one or more substituents independently chosen from a halogen atom, a (C₁-C₄)alkyl and a trifluoromethyl group;
• an indolyl, which is unsubstituted or substituted one or more times with substituents independently chosen from a halogen atom and a (C₁-C₄)alkyl;
• a benzyofuryl, which is unsubstituted or substituted one or more times with substituents independently chosen from a halogen atom and a (C₁-C₄)alkyl;
• a 2,1,3-benzoxadiazolyl;
in the form of base or of acid-addition salts, or in the form of hydrates or solvates.

2. Compound according to Claim 1, of formula (IA) in which -X- represents a group -CO- and the substituents R₁ to R₄ are as defined for the compounds of formula (I) ;
in the form of base or of acid-addition salts, or in the form of hydrates or solvates.

3. Compound according to Claim 1, of formula (IB) in which -X- represents a group -SO₂- and the substituents R₁ to R₄ are as defined for the compounds of formula (I) ;
in the form of base or of acid-addition salts, or in the form of hydrates or solvates.

4. Compound according to Claim 1, of formula (IC) in which -X- represents a group -CON(R₅)- and the substituents R₁ to R₅ are as defined for the compounds of formula (I);
in the form of base or of acid-addition salts, or in the form of hydrates or solvates.

5. Compound according to Claim 1, of formula (ID) in which -X- represents a group -CSN(R₅)- and the substituents R₁ to R₅ are as defined for the compounds of formula (I);
in the form of base or of acid-addition salts, or in the form of hydrates or solvates.

6. Process for preparing a compound of formula (I) according to Claim 1, **characterized in that**:
a compound of formula: 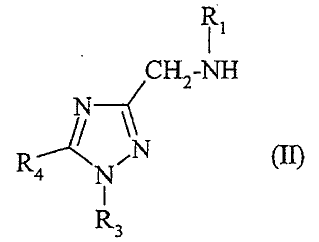 in which R₁, R₃ and R₄ are as defined for a compound of formula (I) of Claim 1 is treated:
- either with an acid or a functional derivative of this acid of formula:
HOOC-R₂ (III)
in which R₂ is as defined for a compound of formula (I) in Claim 1, when a compound of formula (IA) needs to be prepared, in which -X- represents a group -CO-;
- or with a sulfonyl halide of formula:
Hal-SO₂-R₂ (IV)
in which R₂ is as defined for a compound of formula (I) in Claim 1 and Hal represents a halogen atom, preferably chlorine, when a compound of formula (IB) needs to be prepared, in which -X- represents a group - SO₂-;
- or with a haloformate of formula:
HalCOOAr (V)
in which Hal represents a halogen atom and Ar represents a phenyl or a 4-nitrophenyl, to obtain an intermediate compound of formula: 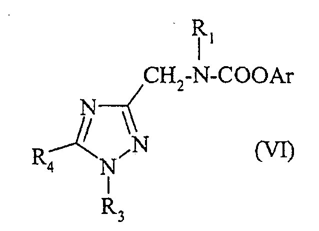 in which R₁, R₃ and R₄ are as defined for a compound of formula (I) in Claim 1, which is then reacted with an amine of formula:
HN(R₅)R₂ (VII)
in which R₂ and R₅ are as defined for a compound of formula (I) in Claim 1, when a compound of formula (IC) needs to be prepared, in which -X- represents a group -CON(R₅)-;
- or with an isothiocyanate R₂N=C=S (VIII), when a compound of formula (ID) needs to be prepared, in which -X- represents a group -CSNH-.

7. Medicament, **characterized in that** it comprises a compound of formula (I) according to any one of Claims 1 to 5, or a hydrate or solvate of a compound of formula (I).

8. Pharmaceutical composition, **characterized in that** it comprises a compound of formula (I) according to any one of Claims 1 to 5, or a hydrate or solvate of this compound, and also at least one pharmaceutically acceptable excipient.

9. Use of a compound of formula (I) according to any one of Claims 1 to 5, for the preparation of a medicament for treating and preventing appetite disorders, metabolic disorders, gastrointestinal disorders, inflammatory phenomena, immune system diseases, psychotic disorders, alcohol dependency and nicotine dependency.

## Patentansprüche

1. Verbindung der Formel: 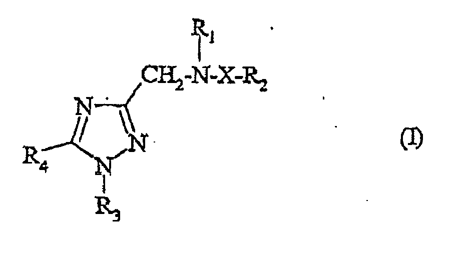 worin:
- X für eine Gruppe 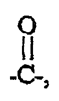 -SO₂-, 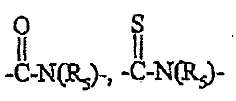 steht;
- R₁ für ein Wasserstoffatom oder eine (C₁-C₄)-Alkylgruppe steht;
- R₂ für:
. (C₁-C₇)-Alkyl, das gegebenenfalls durch eine Trifluormethylgruppe oder eine CO₂R₆- oder CONR₇R₈-Gruppe substituiert ist;
. einen nichtaromatischen carbocyclischen C₃-C₁₂-Rest, der gegebenenfalls ein- oder mehrfach durch (C₁-C₄)-Alkyl substitutiert ist;
. Methyl, das durch einen nichtaromatischen carbocyclischen C₃-C₁₂-Rest substituiert ist und am Carbocyclus gegebenenfalls ein- oder mehrfach durch (C₁-C₄)-Alkyl substituiert ist;
. Phenyl, das gegebenenfalls ein- oder mehrfach durch Substituenten substituiert ist, die unabhängig voneinander unter einem Halogenatom, (C₁-C₄)-Alkyl, Hydroxyl, (C₁-C₄)-Alkoxy, Cyano, einer Trifluormethylgruppe, einer Trifluormethoxygruppe, einer CO₂R₆- oder CONR₇R₈-Gruppe oder einem Phenyl-, Phenoxy-, Pyrrolyl-, Imidazolyl-, Pyridyl- oder Pyrazolylrest, wobei diese Reste gegebenenfalls ein- oder mehrfach durch (C₁-C₄)-Alkyl substituiert sind, ausgewählt sind;
. Phenoxymethyl, das gegebenenfalls am Methyl durch eine oder zwei (C₁-C₄)-Alkylgruppen substituiert ist und gegebenenfalls am Phenyl durch einen oder mehrere unabhängig voneinander unter einem Halogenatom, (C₁-C₄)-Alkyl, (C₁-C₄)-Alkoxy, einer Trifluormethylgruppe, einer Trifluormethoxygruppe, einer CO₂R₆- oder CONR₇R₈-Gruppe ausgewählte Substituenten substituiert ist;
. Benzyl, das gegebenenfalls am Phenyl ein- oder mehrfach durch Substituenten substituiert ist, die unabhängig voneinander unter einem Halogenatom, Cyano, (C₁-C₄)-Alkyl, (C₁-C₄)-Alkoxy, einer Trifluormethylgruppe, einer Trifluormethoxygruppe, einer CO₂R₆- oder CONR₇R₈-Gruppe ausgewählt sind, oder in alpha-Stellung durch eine oder zwei gleiche oder verschiedene Gruppen substituiert ist, die unter (C₁-C₄)-Alkyl und (C₃-C₇)-Cycloalkyl ausgewählt sind;
. eine Benzhydryl- oder Benzhydrylmethylgruppe;
. 1,2,3,4-Tetrahydronaphthalinyl, das gegebenenfalls ein- oder mehrfach durch (C₁-C₄)-Alkyl substituiert ist;
. einen Pyrrolyl-, Imidazolyl-, Pyridyl-, Pyrazolyl-, Furyl- oder Thienylrest, wobei diese Reste gegebenenfalls durch einen oder mehrere Substituenten substituiert sind, die unabhängig voneinander unter einem Halogenatom, einer (C₁-C₄)-Alkylgruppe und einer Trifluormethylgruppe ausgewählt sind;
. Indolyl, das gegebenenfalls ein- oder mehrfach durch Substituenten substituiert ist, die unabhängig voneinander unter einem Halogenatom und (C₁-C₄)-Alkyl ausgewählt sind;
. Benzofuryl, das gegebenenfalls ein- oder mehrfach durch Substituenten substituiert ist, die unabhängig voneinander unter einem Halogenatom und (C₁-C₄)-Alkyl ausgewählt sind;
. 2,1,3-Benzoxadiazolyl
steht;
- R₃ für Phenyl, das gegebenenfalls ein- oder mehrfach durch Substituenten substituiert ist, die unabhängig voneinander unter einem Halogenatom, (C₁-C₄)-Alkyl, (C₁-C₄)-Alkoxy, einer Trifluormethylgruppe, einer Trifluormethoxygruppe und einer S(O)ₙ-Alk-Gruppe ausgewählt sind, steht;
- R₄ für Phenyl, das gegebenenfalls ein- oder mehrfach durch Substituenten substituiert ist, die unabhängig voneinander unter einem Halogenatom, (C₁-C₄)-Alkyl, (C₁-C₄)-Alkoxy, einer Trifluormethylgruppe, einer Trifluormethoxygruppe und einer S(O)ₙ-Alk-Gruppe ausgewählt sind, steht;
- R₅ für ein Wasserstoffatom oder (C₁-C₄)-Alkyl steht;
- R₆ für (C₁-C₄)-Alkyl steht;
- R₇ und R₈ jeweils unabhängig voneinander für ein Wasserstoffatom oder (C₁-C₄)-Alkyl stehen;
- n für 0, 1 oder 2 steht;
- Alk für (C₁-C₄)-Alkyl steht;
mit der Maßgabe, daß dann, wenn X für eine Gruppe -CO- oder -CONR₅- steht, R₂ von:
. einem nichtaromatischen carbocyclischen C₄-C₇-Rest, der gegebenenfalls ein- oder mehrfach durch (C₁-C₄)-Alkyl substitutiert ist;
. Phenyl, das gegebenenfalls ein- oder mehrfach durch Substituenten substituiert ist, die unabhängig voneinander unter einem Halogenatom, (C₁-C₄)-Alkyl, Hydroxyl, (C₁-C₄)-Alkoxy, Cyano, einer Trifluormethylgruppe, einer CO₂R₆- oder CONR₇R₈-Gruppe oder einem Pyrrol-1-yl- oder Pyrazol-1-ylrest, wobei diese Reste gegebenenfalls ein- oder mehrfach durch (C₁-C₄)-Alkyl substituiert sind, ausgewählt sind;
. 1,2,3,4-Tetrahydronaphthalinyl, das gegebenenfalls ein- oder mehrfach durch (C₁-C₄)-Alkyl substituiert ist;
. einem Pyrrolyl-, Imidazolyl-, Pyridyl-, Pyrazolyl-, Furyl- oder Thienylrest, wobei diese Reste gegebenenfalls durch einen oder mehrere Substituenten substituiert sind, die unabhängig voneinander unter einem Halogenatom, einer (C₁-C₄)-Alkylgruppe und einer Trifluormethylgruppe ausgewählt sind;
. Indolyl, das gegebenenfalls ein- oder mehrfach durch Substituenten substituiert ist, die unabhängig voneinander unter einem Halogenatom und (C₁-C₄)-Alkyl ausgewählt sind;
. Benzofuryl, das gegebenenfalls ein- oder mehrfach durch Substituenten substituiert ist, die unabhängig voneinander unter einem Halogenatom und (C₁-C₄)-Alkyl ausgewählt sind;
. 2,1,3-Benzoxadiazolyl
verschieden ist;
in Basen- oder Säureadditionssalzform oder in Hydrat- oder Solvatform.

2. Verbindung nach Anspruch 1 der Formel (IA), worin -X- für eine Gruppe -CO- steht und die Substituenten R₁ bis R₄ die für die Verbindungen der Formel (I) angegebene Bedeutung besitzen;
in Basen- oder Säureadditionssalzform oder in Hydrat- oder Solvatform.

3. Verbindung nach Anspruch 1 der Formel (IB), worin -X- für eine Gruppe -SO₂- steht und die Substituenten R₁ bis R₄ die für die Verbindungen der Formel (I) angegebene Bedeutung besitzen;
in Basen- oder Säureadditionssalzform oder in Hydrat- oder Solvatform.

4. Verbindung nach Anspruch 1 der Formel (IC), worin -X- für eine Gruppe -CON(R₅)- steht und die Substituenten R₁ bis R₅ die für die Verbindungen der Formel (I) angegebene Bedeutung besitzen;
in Basen- oder Säureadditionssalzform oder in Hydrat- oder Solvatform.

5. Verbindung nach Anspruch 1 der Formel (ID), worin -X- für eine Gruppe -CSN(R₅)- steht und die Substituenten R₁ bis R₅ die für die Verbindungen der Formel (I) angegebene Bedeutung besitzen;
in Basen- oder Säureadditionssalzform oder in Hydrat- oder Solvatform.

6. Verfahren zur Herstellung einer Verbindung der Formel (I) nach Anspruch 1, **dadurch gekennzeichnet, daß** man:
eine Verbindung der Formel: 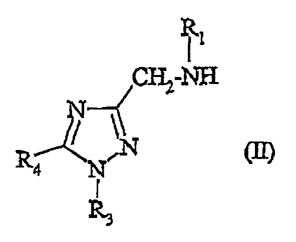 worin R₁, R₃ und R₄ die für eine Verbindung der Formel (I) in Anspruch 1 angegebene Bedeutung besitzen,
- entweder mit einer Säure oder einem funktionellen Derivat dieser Säure der Formel:
HOOC-R₂ (III)
worin R₂ die für eine Verbindung der Formel (I) in Anspruch 1 angegebene Bedeutung besitzt, behandelt, wenn eine Verbindung der Formel (IA), worin -X- für eine Gruppe -CO- steht, hergestellt werden soll;
- oder mit einem Sulfonylhalogenid der Formel:
Hal-SO₂-R₂ (IV)
worin R₂ die für eine Verbindung der Formel (I) in Anspruch 1 angegebene Bedeutung besitzt und Hal für ein Halogenatom, vorzugsweise Chlor, steht, behandelt, wenn eine Verbindung der Formel (IB), worin -X- für eine Gruppe -SO₂- steht, hergestellt werden soll;
- oder mit einem Halogenformiat der Formel:
HalCOOAr (V)
worin Hal für ein Halogenatom steht und Ar für Phenyl oder 4-Nitrophenyl steht, behandelt, wobei man eine Zwischenverbindung der Formel: 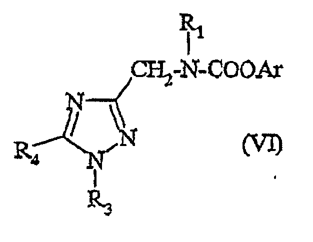 worin R₁, R₃ und R₄ die für eine Verbindung der Formel (I) in Anspruch 1 angegebene Bedeutung besitzen, erhält, die man danach mit einem Amin der Formel:
HN(R₅)R₂ (VII)
worin R₂ und R₅ die für eine Verbindung der Formel (I) in Anspruch 1 angegebene Bedeutung besitzen, umsetzt, wenn eine Verbindung der Formel (IC), worin -X- für eine Gruppe -CON(R₅)- steht, hergestellt werden soll;
- oder mit einem Isothiocyanat R₂N=C=S (VIII) behandelt, wenn eine Verbindung der Formel (ID), worin -X- für eine Gruppe -CSNH- steht, hergestellt werden soll.

7. Arzneimittel, **dadurch gekennzeichnet, daß** es eine Verbindung der Formel (I) nach einem der Ansprüche 1 bis 5 oder ein Hydrat oder ein Solvat einer Verbindung der Formel (I) enthält.

8. Pharmazeutische Zusammensetzung, **dadurch gekennzeichnet, daß** sie eine Verbindung der Formel (I) nach einem der Ansprüche 1 bis 5 oder ein Hydrat oder ein Solvat dieser Verbindung sowie mindestens einen pharmazeutisch unbedenklichen Hilfsstoff enthält.

9. Verwendung einer Verbindung der Formel (I) gemäß einem der Ansprüche 1 bis 5 zur Herstellung eines Arzneimittels zur Behandlung oder Prävention von Appetitstörungen, Stoffwechselstörungen, Magen-Darm-Störungen, Entzündungsphänomenen, Erkrankungen des Immunsystems, psychotischen Störungen, Alkoholabhängigkeit und Nikotinabhängigkeit.
